# FASCICULE DE BREVET EUROPEEN

(11) **EP 1 567 150 B1**
(45) Date de publication et mention de la délivrance du brevet: **12.08.2009**
(21) Numéro de dépôt: 03789510.9
(22) Date de dépôt: 27.11.2003
(51) Int. Cl.: A61K 31/404, C07D 209/42, A61P 25/00, A61P 9/00, A61P 19/00, A61P 37/00, A61P 27/02, A61P 35/00, A61P 17/00, A61P 1/00, A61P 31/00, A61P 5/14

(54) **DERIVES D INDOLE-3-CARBOXAMIDE, LEUR PREPARATION ET LEUR APPLICATION EN THERAPEUTIQUE**
INDOL-3-CARBOXAMIDE, DEREN HERSTELLUNG UND THERAPEUTISCHE VERWENDUNG
DERIVATIVES OF INDOLE-3-CARBOXAMIDE, PREPARATION METHOD THEREOF AND APPLICATION OF SAME IN THERAPEUTICS

(30) Priorité: 29.11.2002 FR 0215086
(43) Date de publication de la demande: 31.08.2005
(73) Titulaire: Sanofi-Aventis, 75013 Paris (FR)
(72) Inventeur: BARTH, Francis, F-34680 Saint-Georges d'Orques (FR); RINALDI-CARMONA, Mireille, F-34680 Saint-Georges-d'Orques (FR); VERNHET, Claude, F-34270 Le Triadou (FR)
(74) Mandataire: Kugel, Dominique
(86) Numéro de dépôt international: PCT/FR2003/003499
(87) Numéro de publication internationale: WO 2004/050086

(56) Documents cités:
- WO-A-01/58869
- WO-A-02/42269

## Description

La présente invention se rapporte à des dérivés d'indole-3-carboxamide, à leur préparation et à leur application en thérapeutique.

Des dérivés de 3-aroylindole de formule : 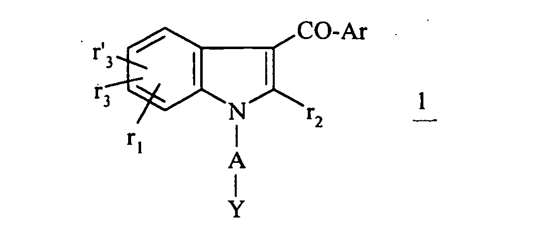 dans laquelle r₁, r₂, r₃, r'₃, A et Y ont différentes valeurs, sont décrits dans la demande de brevet internationale WO 02/42269 ; ces composés sont des agonistes des récepteurs aux cannabinoïdes CB₂.

Des dérivés d'indole-3-carboxamide de formule : 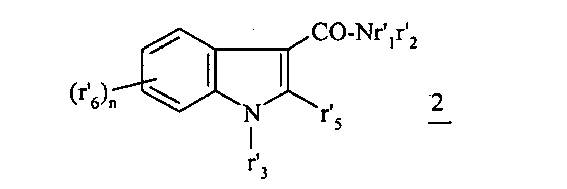 dans laquelle r'₁, r'₂, r'₃, r'₅, r'₆ ont différentes valeurs, sont décrits dans la demande de brevet internationale WO 01/58869. Plus particulièrement, les propriétés modulatrices des récepteurs aux cannabinoïdes CB₂ des dérivés de 7-méthoxy-1-(2-morpholin-4-yléthyl)-1*H*-indole-3-carboxamide sont décrites dans Biorg. Med. Chem. Lett., 2002, 12, 2399-2402.

La présente invention a pour objet des composés répondant à la formule (I) : 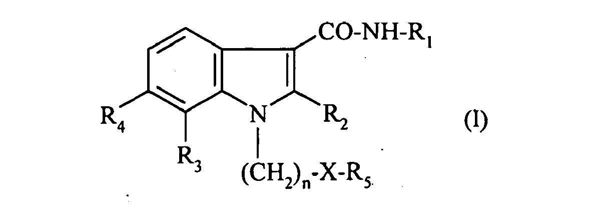 dans laquelle :
- R₁ représente un 1,3,3-triméthylbicyclo[2.2.1]hept-2-yle ou un bicyclo [3.2.1] oct-3-yle ;
- R₂ représente l'hydrogène ou un méthyle ;
- R₃ représente un atome de chlore, de fluor ou de brome ou un groupe méthyle ;
- R₄ représente un atome d'hydrogène, de chlore ou de fluor ou un groupe méthyle ;
- R₅ représente un méthyle ;
- X représente un atome de soufre, un groupe -NHSO₂- ou un groupe -SO₂-;
- n est égal à 2 ou 3.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Les composés de formule (I) peuvent contenir un ou plusieurs atomes de carbone asymétriques. Les différents stéréoisomères ainsi que les racémiques font partie de l'invention.

Dans le cadre de la présente invention, on entend par :
- un atome d'halogène : un fluor, un chlore, un brome ou un iode ;
- un groupe alkyle : un groupe aliphatique saturé linéaire ou ramifié. A titre d'exemple on peut citer les groupes méthyle, éthyle, propyle, isopropyle, butyle, isobutyle, tertbutyle, n-pentyle, isopentyle, n-hexyle, 1,1-diméthylpropyle, 2,2-diméthylpropyle ;
- un groupe carbocyclique en C₅-C₁₀ : un radical monocyclique ou un radical di ou tricyclique condensé ou ponté ; par radical monocyclique on entend un cycloalkyle tel que par exemple cyclopentyle ou cyclohexyle ; par radical di ou tricyclique condensé ou ponté on entend par exemple le bicyclo[2.2.1]heptyle, le bicyclo[3.2.1]octyle, l'adamantyle.

Parmi les composés de formule (I) on distingue les composés de formule (Ia) dans laquelle X représente -NHSO₂-, les composés de formule (Ib) dans laquelle X représente -SO₂- et les composés de formule (Ic) dans laquelle X représente un atome de soufre.

Parmi les composés objet de l'invention, on peut citer les composés préférés qui se définissent comme suit :
- R₁ représente un 1,3,3-triméthylbicyclo[2.2.1]hept-2-yle ou un bicyclo[3.2.1]oct-3-yle ;
- R₂ représente l'hydrogène ou un méthyle ;
- R₃ représente un atome de chlore, de fluor ou de brome ou un groupe méthyle ;
- R₄ représente un atome d'hydrogène, de chlore ou de fluor ou un groupe méthyle ;
- R₅ représente un méthyle ;
- n est égal à 3.

Parmi les composés objets de l'invention, on peut notamment citer les composés suivants :
7-chloro-2-méthyl-1-{3-[(méthylsulfonyl)amino]propyl}-N-(1,3,3-triméthylbicyclo[2.2.1]hept-2-yl)-1*H*-indole-3-carboxamide.
6,7-dichloro-2-méthyl-1-{3-[(méthylsulfonyl)amino]propyl }-N-( 1,3,3-triméthylbicyclo[2.2.1]hept-2-yl)-1*H*-indole-3-carboxamide.
7-bromo-2-méthyl-1-{3-[(méthylsulfonyl)amino]propyl}-N-(1,3,3-triméthylbicyclo[2.2.1]hept-2-yl)-1*H*-indole-3-carboxamide.
7-fluoro-2-méthyl-1-{3-[(méthylsulfonyl)amino]propyl}-N-(1,3,3-triméthylbicyclo[2.2.1]hept-2-yl)-1*H*-indole-3-carboxamide.
6,7-difluoro-2-méthyl-1-{3-[(méthylsulfonyl)amino]propyl}-N-(1,3,3-triméthylbicyclo[2.2.1]hept-2-yl)-1*H*-indole-3-carboxamide.
7-chloro-2,6-diméthyl-1-{3-[(méthylsulfonyl)amino]propyl}-N-(1,3,3-triméthylbicyclo[2.2.1]hept-2-yl)-1*H*-indole-3-carboxamide.
7-bromo-2,6-diméthyl-1-{3-[(méthylsulfonyl)amino]propyl}-N-(1,3,3-triméthylbicyclo[2.2.1]hept-2-yl)-1*H*-indole-3-carboxamide.
7-fluoro-2,6-diméthyl-1-{3-[(méthylsulfonyl)amino]propyl}-N-(1,3,3-triméthylbicyclo[2.2.1]hept-2-yl)-1*H*-indole-3-carboxamide.
7-chloro-2-méthyl-1-{3-[(méthylsulfonyl)amino]éthyl}-N-(1,3,3-triméthylbicyclo[2.2.1]hept-2-yl)-1*H*-indole-3-carboxamide.
6,7-dichloro-2-méthyl-1-{3-[(méthylsulfonyl)amino]éthyl}-N-(1,3,3-triméthylbicyclo[2.2.1]hept-2-yl)-1*H*-indole-3-carboxamide.
7-bromo-2-méthyl-1-{3-[(méthylsulfonyl)amino]éthyl}-N-(1,3,3-triméthylbicyclo[2.2.1]hept-2-yl)-1*H*-indole-3-carboxamide.
7-fluoro-2-méthyl-1-{3-[(méthylsulfonyl)amino]éthyl}-N-(1,3,3-triméthylbicyclo[2.2.1]hept-2-yl)-1*H*-indole-3-carboxamide.
6,7-difluoro-2-méthyl-1-{3-[(méthylsulfonyl)amino]éthyl}-N-(1,3,3-triméthylbicyclo[2.2.1]hept-2-yl)-1*H*-indole-3-carboxamide.
7-chloro-2,6-diméthyl-1-{3-[(méthylsulfonyl)amino]éthyl}-N-(1,3,3-triméthylbicyclo[2.2.1]hept-2-yl)-1*H*-indole-3-carboxamide.
7-bromo-2,6-diméthyl-1- {3-[(méthylsulfonyl)amino]éthyl} -N-(1,3,3-triméthylbicyclo[2.2.1]hept-2-yl)-1*H*-indole-3-carboxamide.
7-fluoro-2,6-diméthyl-1-{3-[(méthylsulfonyl)amino]éthyl}-N-(1,3,3-triméthylbicyclo[2.2.1]hept-2-yl)-1*H*-indole-3-carboxamide.
7-chloro-2-méthyl-1-{3-(méthylsulfonyl)propyl}-N-(1,3,3-triméthyl bicyclo[2.2.1]hept-2-yl)-1*H*-indole-3-carboxamide.
6,7-dichloro-2-méthyl-1-{3-(méthylsulfonyl)propyl }-N-(1,3,3-triméthyl bicyclo[2.2.1]hept-2-yl)-1*H-*indole-3-carboxamide.
7-bromo-2-méthyl-1-{3-(méthylsulfonyl)propyl} -N-(1,3,3-triméthyl bicyclo[2.2.1]hept-2-yl)-1*H*-indole-3-carboxamide.
7-fluoro-2-méthyl-1-{3-(méthylsulfonyl)propyl} -N-(1,3,3-triméthyl bicyclo[2.2.1]hept-2-yl)-1*H*-indole-3-carboxamide.
6,7-difluoro-2-méthyl-1- {3-(méthylsulfonyl)propyl }-N-( 1,3,3-triméthyl bicyclo[2.2.1]hept-2-yl)-1*H*-indole-3-carboxamide.
7-chloro-2,6-diméthyl-1-{3-(méthylsulfonyl)propyl}-N-(1,3,3-triméthyl bicyclo[2.2.1]hept-2-yl)-1*H*-indole-3-carboxamide.
7-bromo-2,6-diméthyl-1- {3-(méthylsulfonyl)propyl }-N-( 1,3,3-triméthyl bicyclo[2.2.1]hept-2-yl)-1*H*-indole-3-carboxamide.
7-fluoro-2,6-diméthyl-1- {3-(méthylsulfonyl)propyl }-N-( 1,3,3-triméthyl bicyclo[2.2.1]hept-2-yl)-1*H*-indole-3-carboxamide.
7-chloro-2-méthyl-1-{3-(méthylsulfonyl)éthyl}-N-(1,3,3-triméthyl bicyclo[2.2.1]hept-2-yl)-1*H*-indole-3-carboxamide.
6,7-dichloro-2-méthyl-1-{3-(méthylsulfonyl)éthyl}-N-(1,3,3-triméthyl bicyclo[2.2.1]hept-2-yl)-1*H*-indole-3-carboxamide.
7-bromo-2-méthyl-1-{3-(méthylsulfonyl)éthyl }-N-( 1,3,3-triméthyl bicyclo[2.2.1]hept-2-yl)-1*H*-indole-3-carboxamide.
7-fluoro-2-méthyl-1-{3-(méthylsulfonyl)éthyl}-N-(1,3,3-triméthyl bicyclo[2.2.1]hept-2-yl)-1*H*-indole-3-carboxamide.
6,7-difluoro-2-méthyl-1-{3-(méthylsulfonyl)éthyl}-N-(1,3,3-triméthyl bicyclo[2.2.1]hept-2-yl)-1*H*-indole-3-carboxamide.
7-chloro-2,6-diméthyl-1-{3-(méthylsulfonyl)éthyl}-N-(1,3,3-triméthyl bicyclo[2.2.1]hept-2-yl)-1*H*-indole-3-carboxamide.
7-bromo-2,6-diméthyl-1- {3-(méthylsulfonyl)éthyl }-N-( 1,3,3-triméthyl bicyclo[2.2.1]hept-2-yl)-1*H*-indole-3-carboxamide.
7-fluoro-2,6-diméthyl-1-3-(méthylsulfonyl)éthyl }-N-( 1,3,3-triméthyl bicyclo[2.2.1]hept-2-yl)-1*H*-indole-3-carboxamide.
N-bicyclo[3.2.1 ]oct-3-yl-7-chloro-2-méthyl-1- {3-[(méthylsulfonyl)amino]propyl} -1*H*-indole-3-carboxamide.
N-bicyclo[3.2.1 ]oct-3-yl-6,7-dichloro-2-méthyl-1-{3-[(méthylsulfonyl)amino] propyl}-1*H*-indole-3-carboxamide.
N-bicyclo[3.2.1]oct-3-yl-7-bromo-2-méthyl-1-{3-[(méthylsulfonyl)amino] propyl}-1*H*-indole-3-carboxamide.
N-bicyclo[3.2.1]oct-3-yl-7-fluoro-2-méthyl-1-{3-[(méthylsulfonyl)amino]propyl} -1*H*-indole-3-carboxamide.
N-bicyclo[3.2.1]oct-3-yl-6,7-difluoro-2-méthyl-1-{3-[(méthylsulfonyl)amino] propyl}-1*H*-indole-3-carboxamide.
N-bicyclo[3.2.1 ]oct-3-yl-7-chloro-2,6-diméthyl-1-{3-[(méthylsulfonyl)amino] propyl}-1*H*-indole-3-carboxamide.
N-bicyclo[3.2.1]oct-3-yl-7-bromo-2,6-diméthyl-1-{3-[(méthylsulfonyl)amino] propyl}-1*H*-indole-3-carboxamide.
N-bicyclo[3.2.1]oct-3-yl-7-fluoro-2,6-diméthyl-1-{3-[(méthylsulfonyl)amino] propyl}-1*H*-indole-3-carboxamide.
N-bicyclo[3.2.1]oct-3-yl-7-chloro-2-méthyl-1-{3-[(méthylsulfonyl)amino]éthyl} - 1*H*-indole-3-carboxamide.
N-bicyclo[3.2.1]oct-3-yl-6,7-dichloro-2-méthyl-1-{3-[(méthylsulfonyl)amino] éthyl}-1*H*-indole-3-carboxamide.
N-bicyclo[3.2.1]oct-3-yl-7-bromo-2-méthyl-1-{3-[(méthylsulfonyl)amino]éthyl}-1*H*-indole-3-carboxamide.
N-bicyclo[3.2.1]oct-3-yl-7-fluoro-2-méthyl-1-{3-[(méthylsulfonyl)amino]éthyl}-1 *H*-indole-3-carboxamide.
N-bicyclo[3.2.1]oct-3-yl-6,7-difluoro-2-méthyl-1-{3-[(méthylsulfonyl)amino] éthyl}-1*H*-indole-3-carboxamide.
N-bicyclo[3.2.1]oct-3-yl-7-chloro-2,6-diméthyl-1-{3-[(méthylsulfonyl)amino] éthyl}-1*H*-indole-3-carboxamide.
N-bicyclo[3.2.1 ]oct-3-yl-7-bromo-2,6-diméthyl-1-{3-[(méthylsulfonyl)amino] éthyl}-1*H*-indole-3-carboxamide.
N-bicyclo[3.2.1]oct-3-yl-7-fluoro-2,6-diméthyl-1-{3-[(méthylsulfonyl)amino] éthyl}-1*H*-indole-3-carboxamide.
N-bicyclo[3.2.1 ]oct-3-yl-7-chloro-2-méthyl-1-{3-(méthylsulfonyl)propyl}-1 *H-*indole-3-carboxamide.
N-bicyclo[3.2.1]oct-3-yl-6,7-dichloro-2-méthyl-1-{3-(méthylsulfonyl)propyl}-1 *H*-indole-3-carboxamide.
N-bicyclo[3.2.1 ]oct-3-yl-7-bromo-2-méthyl-1-{3-(méthylsulfonyl)propyl} -1*H-*indole-3-carboxamide.
N-bicyclo[3.2.1]oct-3-yl-7-fluoro-2-méthyl-1-{3-(méthylsulfonyl)propyl}-1 *H-*indole-3-carboxamide.
N-bicyclo[3.2. 1]oct-3-yl-6,7-difluoro-2-méthyl-1-{3-(méthylsulfonyl)propyl)-1 *H*-indole-3-carboxamide.
N-bicyclo[3.2.1]oct-3-yl-7-chloro-2,6-diméthyl-1-{3-(méthylsulfonyl)propyl}-1*H*-indole-3-carboxamide.
N-bicyclo[3.2.1]oct-3-yl-7-bromo-2,6-diméthyl-1-{3-(méthylsulfonyl)propyl}-1 *H*-indole-3-carboxamide.
N-bicyclo[3.2. ]oct-3-yl-7-fluoro-2,6-diméthyl-1-{3-(méthylsulfonyl)propyl}-1*H*-indole-3-carboxamide.
N-bicyclo[3.2.1]oct-3-yl-7-chloro-2-méthyl-1-{3-(méthylsulfonyl)éthyl}-1*H-*indole-3-carboxamide.
N-bicyclo[3.2.1]oct-3-yl-6,7-dichloro-2-méthyl-1-{3-(méthylsulfonyl)éthyl}-1*H-*indole-3-carboxamide.
N-bicyclo[3.2.1]oct-3-yl-7-bromo-2-méthyl-1-{3-(méthylsulfonyl)éthyl}-1*H* indole-3-carboxamide.
N-bicyclo[3.2.1]oct-3-yl-7-fluoro-2-méthyl-1-{3-(méthylsulfonyl)éthyl}-1*H-*indole-3-carboxamide.
N-bicyclo[3.2.1]oct-3-yl-6,7-difluoro-2-méthyl-1-{3-(méthylsulfonyl)éthyl}-1*H-*indole-3-carboxamide.
N-bicyclo[3.2.1]oct-3-yl-7-chloro-2,6-diméthyl-1-{3-(méthylsulfonyl)éthyl}-1*H-*indole-3-carboxamide.
N-bicyclo[3.2.1]oct-3-yl-7-bromo-2,6-diméthyl-1-{3-(méthylsulfonyl)éthyl}-1 *H-*indole-3-carboxamide.
N-bicyclo[3.2.1]oct-3-yl-7-fluoro-2,6-diméthyl-1-(3-(méthylsulfonyl)éthyl}- 1*H-*indole-3-carboxamide.
7-chloro-2-méthyl-1-(3-(méthylthio)propyl)-N-(1,3,3-triméthylbicyclo[2.2.1]hept-2-yl)-1*H*-indole-3-carboxamide.
7-chloro-1-(3-((méthylsulfonyl)amino)propyl)-N-(1,3,3-triméthylbicyclo[2.2.1]hept-2-yl)-1*H*-indole-3-carboxamide.
6,7-dichloro-1-(3-(méthylthio)propyl)-N-(1,3,3-triméthylbicyclo [2.2.1]hep-2-yl)-1*H*-indole-3-carboxamide.
6,7-dichloro-1-(3-(méthylsulfonyl)propyl)-N-(1,3,3-triméthylbicyclo [2.2.1]hept-2-yl)-1*H*-indole-3-carboxamide.

Tout particulièrement, on préfère les composés suivants :
7-chloro-2-méthyl-1-{3-[(méthylsulfonyl)amino]propyl}-N-(1,3,3-triméthylbicyclo[2.2.1 ]hept-2-yl)-1*H*-indole-3-carboxamide.
6,7-dichloro-2-méthyl-1-{3-[(méthylsulfonyl)amino]propyl }-N-(1,3,3-triméthylbicyclo[2.2.1 ]hept-2-yl)-1 *H*-indole-3-carboxamide.
7-chloro-2-méthyl-1-(3-(méthylthio)propyl)-N-(1,3,3-triméthylbicyclo[2.2.1]hept-2-yl)-1*H*-indole-3-carboxamide.

Corformément à l'invention, on peut préparer les composés de formule générale (I) selon le procédé qui suit.

Ce procédé est caractérisé en ce que on traite un dérivé fonctionnel d'acide indole-3-carboxylique de formule : 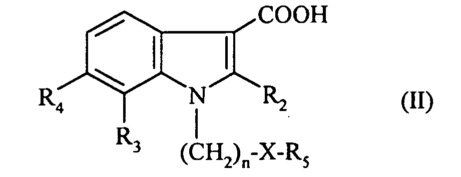 dans laquelle R₂, R₃, R₄, R₅, X et n sont tels que définis pour (I), par une amine de formule R₁NH₂ (III).

Par dérivé fonctionnel d'un acide de formule (II), on entend un chlorure d'acide, un anhydride ou encore l'acide opportunément activé par exemple avec l'hexafluoro phosphate de benzotriazol-1-yloxytris(diméthylamino)phosphonium (BOP) ou l'hexafluorophosphate de (1-benzotriazolyl)oxytris(pyrrolidino)phosphonium (PyBOP).

La réaction est effectuée dans un solvant aprotique tel que le dichlorométhane, le THF ou le DMF, en milieu basique, et en présence d'un agent de couplage tel que le BOP ou la dicyclohexylcarbodiimide (DCC).

Un composé de formule (I) dans laquelle X représente un groupement SO₂ peut être préparé à partir d'un composé de formule (I) portant les mêmes substituants et dans lequel X est un atome de soufre, par action d'un agent oxydant tel que l'acide métachloroperbenzoïque (MCPBA).

Les dérivés d'acide indole-3-carboxylique de formule (II) sont préparés à partir de dérivés indoliques correspondants de formule : 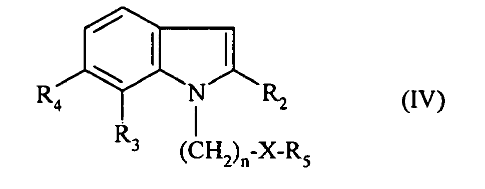 dans laquelle R₂, R₃, R₄, R₅, X et n sont tels que définis pour (I), par action d'un agent acylant. L'agent acylant peut être, par exemple, le chlorure d'oxalyle, ou encore le chlorure de trichloroacétyle. Dans ce dernier cas, le dérivé trichloroacétyle intermédiaire obtenu est ensuite traité successivement par une base forte puis un acide pour conduire à l'acide désiré.

Les dérivés indoliques de formule (IV) sont connus ou préparés par des procédés tels que ceux décrits dans WO 02/42269.

Les composés de formule (II) sont nouveaux et constituent un aspect ultérieur de la présente invention.

Ainsi la présente invention a également pour objet les composés de formule : 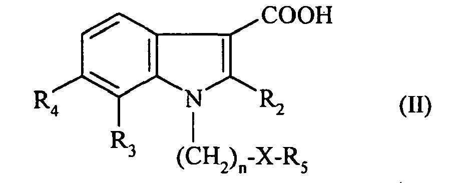 dans laquelle :
- R₂ représente l'hydrogène ou un méthyle ;
- R₃ représente un atome de chlore, de fluor ou de brome ou un groupe méthyle ;
- R₄ représente un atome d'hydrogène, de chlore ou de fluor ou un groupe méthyle ;
- R₅ représente un méthyle ;
- X représente un atome de soufre, un groupe -NHSO₂- ou un groupe -SO₂-;
- n est égal à 2 ou 3.

Les sels et esters alkyliques en C₁-C₆ ou l'ester benzylique des composés de formule (II) font également partie de l'invention.

On préfère les composés de formule (II) dans laquelle :
- R₂ représente un atome d'hydrogène ou un groupe méthyle ;
- R₃ représente un atome de chlore ou de brome ;
- R₄ représente un atome d'hydrogène ou de chlore ou un groupe méthyle ;
- R₅ représente un groupe méthyle ;
- X représente un atome de soufre ou un groupe NHSO₂ ;
- n est égal à 3 ;
ainsi que leurs sels et esters alkyliques en (C₁ -C₆) ou ester benzylique.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans les exemples, on utilise les abréviations suivantes :
TA : température ambiante
DCM : dichlorométhane
THF : tétrahydrofurane
TCE : 1,1,2,2-tétrachloroéthane.

Les spectres de résonance magnétique nucléaire sont enregistrés à 200 MHz dans le DMSO-d₆. Pour l'interprétation des spectres, on utilise les abréviations suivantes : s : singulet, d : doublet, t : triplet, m : massif, mt : multiplet, se : singulet élargi, q : quatruplet, qt : quintuplet.

Les composés selon l'invention sont analysés par couplage LC/UV/MS (chromatographie liquide/détection UV/spectrométrie de masse). On mesure le pic moléculaire (MH⁺) et le temps de rétention (t) en minutes.

On utilise une colonne Symetry Waters^{®} MS C18, commercialisée par Waters, de 2,1 x 50 mm, 3,5 µm, à température ambiante, débit 0,4 mL/minute.

L'éluant est composé comme suit :
- solvant A : 0,005 % d'acide trifluoroacétique (TFA) dans l'eau
- solvant B : 0,005 % de TFA dans l'acétonitrile.

Gradient : Le pourcentage de solvant B varie de 0 à 90 % en 10 minutes avec un plateau à 90 % de B pendant 5 minutes.

On injecte 1 µl de produit à analyser à la concentration de 1 mg/ml.

La détection UV est effectuée à 210 nm et la détection de masse en mode ionisation chimique à pression atmosphérique.

### EXEMPLE 1 : composé 1

7-Chloro-2-méthyl-1-{3-[(méthylsulfonyl)amino]propyl}-N-[(l 1S)-endo-(1,3,3-triméthylbicyclo[2.2.1]hept-2-yl)]-1*H*-indole-3-carboxamide.

### A) Acide 7-chloro-2-méthyl-1-(3-((méthylsulfonyl)amino)propyl)-1H-indole-3-carboxylique.

Sous azote, on dissout 2 g de 7-chloro-2-méthyl-1-[3-(méthylsulfonylamino) propyl]indole dans 16 ml de TCE à TA et on ajoute 0,645 ml de chlorure d'oxalyle dans 7 ml de TCE. On chauffe à 120°C pendant 16 heures puis on refroidit à TA. Le milieu réactionnel est dilué par addition de 20 ml d'eau puis on le verse sur 50 ml d'une solution d'HCl à 10 %. On décante puis on lave la phase aqueuse par du DCM. Les phases organiques sont réunies puis lavées par une solution saturée de NaCl. On sèche sur Na₂SO₄ puis on concentre à sec et on purifie par chromatographie sur silice en éluant par CH₂Cl₂-MeOH (95/5 ; v/v). On obtient 1,33 g du composé attendu.

RMN : 1,8-2,0 ppm : m : 2H ; 2,7 ppm : s : 3H ; 2,9 ppm : s : 3H ; 3,1 ppm : q : 2H ; 4,5 ppm : t.e. : 2H ; 7,1-7,3 ppm : m : 3H ; 8,1 ppm : d : 1H.

### B) (7-Chloro-2-méthyl-1-{3-[(méthylsulfonyl)amino]propyl}-N-[(1S)-endo-(1,3,3-triméthylbicyclo[2.2.1]hept-2-yl)]-1H-indole-3-carboxamide.

Sous azote, on introduit 0,63 g du composé de l'étape précédente dans 35 ml de DCM et on ajoute successivement à TA 0,35 g de chlorhydrate de (1S)-endo-1,1,3-triméthylbicyclo[2.2.1]heptan-2-ylamine. 0,765 ml de triéthylamine et 0,95 g d'hexafluorophosphate de (1-benzotriazolyl)oxytris(pyrrolidino)phosphonium (PyBOP). On laisse sous agitation pendant 2 heures 15 minutes à TA et on contrôle par CCM qu'il n'y a plus de produit de départ. On verse le milieu réactionnel dans de l'eau puis on extrait successivement par une solution de KHSO₄/K2SO₄, par de l'eau par une solution de soude à 10 % puis par une solution saturée de NaCl. On sèche sur Na₂SO₄ et évapore les solvants puis on chromatographie une première fois sur silice en éluant par un mélange AcOEt/cyclohexane (7/3 ; v/v). Le produit obtenu est chromatographié à nouveau sur silice en éluant par AcOEt/cyclohexane (7/3 ; v/v). On obtient 230 mg de produit, F = 134-136°C.

RMN : 0,8-2,1 ppm : m : 18H ; 2,6 ppm : s : 3H ; 2,9 ppm : s : 3H ; 3,1 ppm : q : 2H ; 3,7 ppm : d : 1H ; 4,5 ppm : t.e. : 2H ; 7,0-7,3 ppm : m : 4H ; 7,6 ppm : d : 1H.

### EXEMPLE 2: composé 6

7-Chloro-2-méthyl-1-(3-(méthylthio)propyl)-N-(1,3,3-triméthylbicyclo[2.2.1] hept-2-yl)-1*H*-indole-3-carboxamide.

### A) Acide 7-chloro-2-méthyl-1-(3-((méthylthio)propyl)-1H-indole-3-carboxylique.

Sous azote on dissout 1,58 g de 7-chloro-2-méthyl-1-(3-(méthylthio)propyl)indole dans 16 ml de TCE à TA. On ajoute 0,602 ml de chlorure d'oxalyle dans 8 ml de TCE et on agite à 120°C pendant 16 heures. On concentre à sec, puis on traite par 125 ml d'EtOH et 3,5 g de KOH à 80°C pendant 16 heures. On concentre à sec, reprend par AcOEt/H₂O, on lave la phase organique par HCl 10 %, puis NaCl saturé. On sèche sur MgSO₄ et concentre. On obtient 1 g du composé attendu.

RMN : 1,9-2,2 ppm : m : 5H ; 2,6 ppm : t : 2H ; 2,8 ppm : s : 3H ; 4,6 ppm : m : 2H ; 7,1-7,3 ppm : m : 2H ; 8,1 ppm : d : 1H ; 12,3 ppm : s : 1H.

### B) 7-Chloro-2-méthyl-1-(3-(méthylthio)propyl)-N-(1,3,3-triméthylbicyclo[2.2.1] hept-2-yl)-1H-indole-3-carboxamide.

On mélange 0,94 g de l'acide obtenu à l'étape précédente avec 1,32 ml de triéthylamine, 0,6 g de chlorhydrate de fenchylamine et 1,64 g de PyBOP dans 40 ml de TCE. On porte à 100°C pendant 1 heure. On dilue avec du DCM et lave par un tampon KHSO₄/K₂SO₄. On extrait les phases organiques successivement par H₂O, une solution de NaOH à 10 % et une solution saturée de NaCl puis on sèche sur MgSO₄ et concentre à sec. On chromatographie sur silice le produit brut obtenu en éluant par le mélange cyclohexane/acétate d'éthyle (4/1 ; v/v). Après une deuxième chromatographie en éluant par DCM/MeOH (95/5 ; v/v), on obtient 0,8 g du composé attendu.

### EXEMPLE 3: composé 7

7-Chloro-2-méthyl-1-{3-[(méthylsulfonyl)amino]éthyl}-N-(1,3,3-triméthylbicyclo [2.2.1]hept-2-yl)-1*H*-indole-3-carboxamide.

On introduit 1,06 g d'acide métachloroperbenzoïque (MCPBA) dans 30 ml de DCM, on refroidit à + 5°C et on ajoute 0,73 g du composé obtenu à l'exemple précédent dans 30 ml de DCM. On laisse le milieu revenir à TA puis on agite 2 heures et demie. On verse le milieu réactionnel sur une solution de NaOH à 30 %, on lave deux fois la phase aqueuse par du DCM, puis on extrait les phases organiques réunies par NaOH à 10 %, H₂O, puis NaCl saturé. On sèche sur MgSO₄ et concentre à sec. Deux chromatographies successives sur silice en éluant par du cyclohexane/acétate d'éthyle (3/2 ; v/v) permettent d'obtenir 80 mg de produit attendu, F = 122°C.

### EXEMPLE 4: composé 4

6,7-Dichloro-2-méthyl-1-{3-[(méthylsulfonyl)amino]propyl}-N-(1,3,3-triméthylbicyclo[2.2.1]hept-2-yl)-1*H*-indole-3-carboxamide.

### A) Acide 6,7-dichloro-2-méthyl-1-(3-((méthylsulfonyl)amino)propyl)-1H-indole-3-carboxylique.

Sous azote on dissout 1,87 g de 6,7-dichloro-2-méthyl-1-(3-((méthylsulfonyl) amino)propyl)indole dans 14 ml de DCE. On ajoute goutte à goutte 0,54 ml de chlorure d'oxalyle dilué par 7 ml de DCE et on laisse agiter 2 heures à TA. On porte ensuite à 120°C pendant 16 heures. On concentre à sec et reprend par une solution de 3 g de KOH dans 120 ml d'EtOH et 2 ml d'eau. On porte à reflux 16 heures. On concentre à sec et reprend par un mélange AcOEt/H₂O. La phase organique est extraite 3 fois par H₂O, puis les phases aqueuses réunies sont diluées par AcOEt et acidifiées par HCl 35 %. On lave la phase organique par H₂O, puis NaCl saturé. On sèche sur MgSO₄ et concentre à sec. On obtient 0,85 g du composé attendu.

RMN : 1,7-2,0 ppm : qt : 2H ; 2,7 ppm : s : 3H ; 2,85 ppm : s : 3H ; 3,0 ppm : q : 2H ; 4,4-4,6 ppm : m : 2H ; 7,1 ppm : t : 1H ; 7,3 ppm : d : 1H ; 7,9 ppm : d : 1H ; 12,3 ppm : s : 1H.

### B) 6,7-Dichloro-2-méthyl-1-{3-[(méthylsulfonyl)amino]propyl}-N-(1,3,3-triméthylbicyclo[2.2.1]hept-2-yl)-1H-indole-3-carboxamide.

On mélange 0,84 g de l'acide obtenu à l'étape précédente avec 0,925 ml de triéthylamine, 0,42 g de chlorhydrate de fenchylamine, et 1,15 g de PyBOP dans 35 ml de DCE. On porte 1 heure à 100°C, on dilue par DCM et on lave avec du tampon KHSO₄/K₂SO₄, puis H₂O, NaOH 10 %, et enfin NaCl. On sèche sur MgSO₄, on concentre à sec. Le produit brut obtenu est chromatographié sur silice en éluant par un mélange DCM/MeOH (99/1 ; v/v). On obtient le composé attendu 0,52 g, F = 150-154°C.

En procédant comme décrit à l'Exemple 1, étape A (Préparation 1) à l'Exemple 2, étape A (Préparation 2) et à l'Exemple 4, étape A (Préparation 3), on a préparé les acides intermédiaires de formule (II) décrit dans le Tableau ci-après :

**TABLEAU 1**

| | | | | | |
|---|---|---|---|---|---|
| 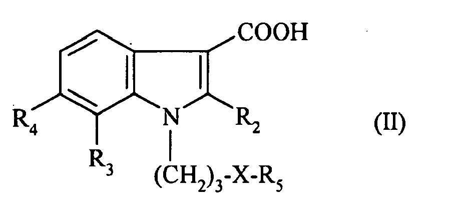 | | | | | |

| Préparations | R₂ | R₃ | R₄ | X-R₅ | Caractérisation |
|---|---|---|---|---|---|
| 4 | Me | Br | Me | NHSO₂Me | RMN : 1,8-2,0 ppm : m : 2H ; 2,4 ppm : s : 3H ; 2,75 ppm : s : 3H ; 2,9 ppm : s : 3H ; 3,1 ppm : q : 2H ; 4,6 ppm : m : 2H ; 7,1 ppm : m : 2H ; 7,9 ppm : d : 1H; 12,1 ppm: s: 1H. |
| 5 | H | Cl | H | NHSO₂Me | RMN: 2,0 ppm: qt: 2H; 2,9 ppm: s : 3H ; 2,95 ppm : q : 2H ; 4,6 ppm : t : 2H ; 7,0-7,2 ppm : m : 2H ; 7,3 ppm : d : 1H ; 8,05 ppm : d : 1H ; 8,15 ppm : s: 1H; 12,2 ppm: s: 1H. |
| 6 | H | Cl | Cl | SMe | RMN : 2-2,2 ppm : m : 5H ; 2,4-2,6 ppm : m : 2H ; 4,6 ppm : t : 2H ; 7,4 ppm : d : 1H ; 8,05 ppm : d : 1H ; 8,2 ppm : s : 1H ; 12,4 ppm : s : 1H. |

Le tableau qui suit illustre les structures chimiques et les propriétés de quelques exemples de composés selon l'invention. Dans ce tableau, Me représente un groupe méthyle, t-Bu un groupe t-butyle.

**TABLEAU 2**

| | | | | | | |
|---|---|---|---|---|---|---|
| 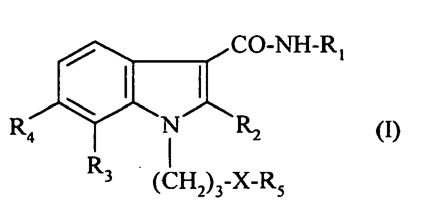 | | | | | | |

| Composés | R₁ | R₂ | R₃ | R₄ | -XR₅ | Caractérisation |
|---|---|---|---|---|---|---|
| 1 | 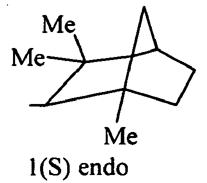 | Me | Cl | H | NHSO₂Me | RMN |
| 2 | 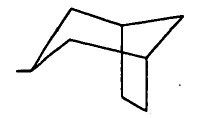 | Me | Cl | H | NHSO₂Me | MH+ = 452,2 t=8,81 |
| 4 | 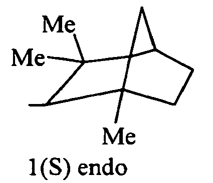 | Me | Cl | Cl | NHSO₂Me | F = 150-154°C |
| 5 | 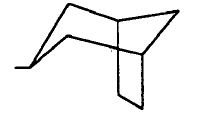 | Me | Br | Me | NHSO₂Me | F = 191-193°C |
| 6 | 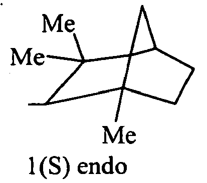 | Me | Cl | H | SMe | MH⁺ = 433,3 t = 11,66 |
| 7 | 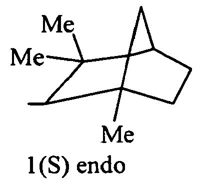 | Me | Cl | H | SO₂Me | F = 122°C |
| 8 | 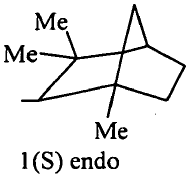 | H | Cl | H | NHSO₂Me | F = 161-162°C |
| 9 | 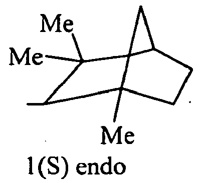 | H | Cl | Cl | SMe | F = 62°C |
| 10 | 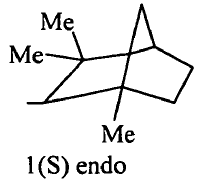 | H | Cl | Cl | SO₂Me | F = 89-92°C |

Les composés selon l'invention ont fait l'objet d'essais pharmacologiques.

Les composés selon l'invention ont montré une bonne affinité *in vitro* pour les récepteurs aux cannabinoïdes (CB₂) et une affinité *in vitro* nettement plus faible pour les récepteurs aux cannabinoïdes (CB₁) qu'il s'agisse de récepteurs humains ou de récepteurs de rongeur. Les essais de liaison par affinité (binding) ont été réalisés selon les conditions expérimentales décrites par Devane et al. (Molecular Pharmacology, 1988, 34, 605-613), avec des membranes issues de lignée cellulaires dans lesquelles les récepteurs CB₁ (Matsuda et al., Nature 1990, 346, 561-564) et CB₂ (Munro et al., Nature 1993, 365, 61-65) ont été exprimés. Pour les récepteurs humains, l'affinité *in vitro* aux cannabinoïdes CB₂ exprimé sous forme de Ki (constante d'inhibition) est inférieure à 10⁻⁷ M et le rapport entre l'affinité pour les récepteurs CB₁ et celle pour les récepteurs CB₂ est d'au moins 100.

D'autre part les composés selon l'invention se comportent *in vitro* comme des agonistes spécifiques des récepteurs humains aux cannabinoïdes CB₂ versus CB₁, ils diminuent la production d'AMPc dans les cellules stimulées par de la forskoline et ce en inhibant l'adénylate cyclase. Les essais ont été réalisés selon les conditions expérimentales décrites par Matsuda et al., Nature 1990, 346, 561-564.

Il apparaît donc que les composés selon l'invention ont une activité agoniste sélective de CB₂ des récepteurs aux cannabinoïdes.

Les composés selon l'invention ou leurs sels éventuels possèdent également une affinité *in vivo* pour les récepteurs aux cannabinoïdes CB₂ présents au niveau de la rate de souris lorsqu'ils sont administrés par voie intraveineuse ou orale. Leur activité a été mise en évidence par des expériences de liaison *ex vivo* du [³H]-CP 55940. Les essais ont été réalisés selon les conditions expérimentales décrites par M. Rinaldi-Carmona et al., Life Sciences, 1995, 56, 1941-1947.

Les composés selon l'invention peuvent donc être utilisés pour la préparation de médicaments, en particulier de médicaments agonistes des récepteurs aux cannabinoïdes CB₂.

Ainsi, selon un autre de ses aspects, l'invention a pour objet des médicaments qui comprennent un composé de formule (I) ou un hydrate ou un solvat du composé de formule (I).

Ces médicaments trouvent leur emploi en thérapeutique, notamment dans le traitement et la prévention des affections suivantes :
anémies autoimmunes hémolytiques, sclérose en plaques, sclérose latérale amyotrophique, amyloses, glomérulonéphrite, le rejet de greffe, les maladies affectant la lignée plasmocytaire ; les maladies allergiques: hypersensibilité retardée ou immédiate, rhinite allergique, dermatite de contact, conjonctivite allergique ; les maladies infectieuses parasitaire, virale ou bactérienne : SIDA, méningites ;
les maladies inflammatoires, notamment les maladies articulaires : arthrite, arthrite rhumatoïde, arthrite réactionnelle, ostéoarthrite, spondylite, spondylarthrite ankylosante, spondylarthrite indifférenciée, goutte, vascularite, thyroïdite, maladie de Behcet, maladie de Crohn, maladie du colon irritable syndrome de colon irritable (an anglais IBD : inflammatory bowel disease et IBS : irritable bowel syndrome) ;
l'ostéoporose ; la douleur : les douleurs chroniques de type inflammatoire, les douleurs neuropathiques, les douleurs aigues périphériques ; les affections oculaires : hypertension oculaire, glaucome ; les affections pulmonaires : maladies des voies respiratoires, asthme, bronchite chronique, obstruction chronique des voies respiratoires (en anglais COPD : chronic obstructive pulmonary disease), emphysème; ; les maladies du système nerveux central et les maladies neurogénératives : syndrome de Tourette, maladie de Parkinson, maladie d'Alzheimer, démence sénile, chorée, chorée de Huntington, épilepsie, psychoses, dépression, lésions de la moëlle épinière ; la migraine, les vertiges, les vomissements, les nausées en particulier ceux consécutifs à une chimiothérapie ; les maladies cardiovasculaires en particulier hypertension, artériosclérose, crise cardiaque, ischémie cardiaque ; l'ischémie rénale ; les cancers : les tumeurs bégnines de la peau, papillomes et tumeurs cancéreuses, les cancers de poumon et cancers du poumon à petites cellules, les tumeurs de la prostate, les tumeurs cérébrales (glioblastomes, médullo-epithéliomes, médulloblastomes, neuroblastomes, tumeurs d'origine embryonnaires, astrocytomes, astroblastomes, épendymomes, oligodendrogliomes, tumeur du plexus, neuro-epithéliomes, tumeur de l'épiphyse, épendymoblastomes, neuroectodermique, méningiomes malins, sarcomatoses, mélanomes malins, schwénnomes) ; les maladies gastro-intestinales ; l'obésité, les désordres du système immunitaire, notamment les maladies autoimmunes : psoriasis, lupus érythémateux, maladies du tissu conjonctif ou connectivites, dermatite de contact, syndrome de Sjögrer's, syndrome de Guillain-Barré ; les cirrhoses et les cirrhoses chroniques du foie.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Par voie orale, la dose de principe actif administrée par jour peut atteindre 0,01 à 20 mg/kg, en une ou plusieurs prises.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention ou hydrates ou solvats.

## Revendications

1. Composé répondant à la formule (I) : 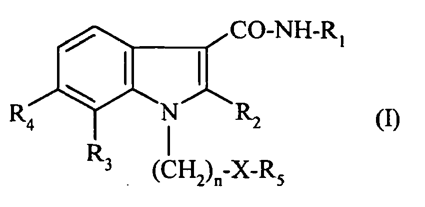 dans laquelle :
- R₁ représente un 1,3,3-triméthylbicyclo[2.2.1]hept-2-yle ou un bicyclo [3.2.1] oct-3-yle ;
- R₂ représente l'hydrogène ou un méthyle ;
- R₃ représente un atome de chlore, de fluor ou de brome ou un groupe méthyle ;
- R₄ représente un atome d'hydrogène, de chlore ou de fluor ou un groupe méthyle ;
- R₅ représente un méthyle ;
- X représente un atome de soufre, un groupe -NHSO₂- ou un groupe -SO₂-;
- n est égal à 2 ou 3 ;
ainsi qu'à l'état d'hydrate ou de solvat.

2. Composé selon la revendication 1 **caractérisé en ce qu'**il est choisi parmi :
le 7-chloro-2-méthyl-1-{3-[(méthylsulfonyl)amino]propyl}-N-[(1S)-endo-(1,3,3-triméthylbicyclo [2.2.1]hept-2-yl)]-1*H*-indole-3-carboxamide ; le 6,7-dichloro-2-méthyl-1-{3-[(méthylsulfonyl)amino]propyl}-N-(1,3,3-triméthylbicyclo[2.2.1]hept-2-yl)-1*H*-indole-3-carboxamide ; le 7-chloro-2-méthyl-1-(3-(méthylthio)propyl)-N-(1,3,3-triméthylbicyclo[2.2.1]hept-2-yl)-1*H-*indole-3-carboxamide ; ainsi qu'à l'état d'hydrate ou de solvat.

3. Procédé de préparation d'un composé de formule (I) selon l'une quelconque des revendications 1 à 2 **caractérisé en ce que** :
on traite un dérivé fonctionnel d'acide indole-3-carboxylique de formule : 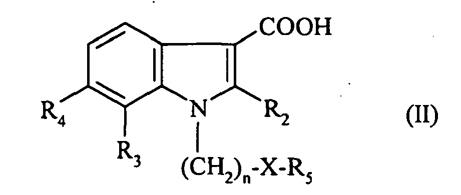
dans laquelle R₂, R₃, R₄, R₅, X et n sont tels que définis pour (I), par une amine de formule R₁NH₂ (III).

4. Composé de formule : 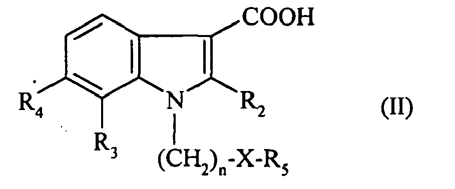 dans laquelle :
- R₂ représente un atome d'hydrogène ou un groupe méthyle ;
- R₃ représente un atome de chlore, de fluor ou de brome ou un groupe méthyle ;
- R₄ représente un atome d'hydrogène, de chlore ou de fluor ou un groupe méthyle ;
- R₅ représente un méthyle ;
- X représente un atome de soufre, un groupe -NHSO₂- ou un groupe -SO₂-;
- n est égal à 2 ou 3 ;
ainsi que leur sels et esters d'alkyles en C₁-C₆ et l'ester benzylique.

5. Composés selon la revendication 4 de formule (II) dans laquelle :
- R₂ représente un atome d'hydrogène ou un groupe méthyle ;
- R₃ représente un atome de chlore ou de brome ;
- R₄ représente un atome d'hydrogène ou de chlore ou un groupe méthyle ;
- R₅ représente un groupe méthyle ;
- X représente un atome de soufre ou un groupe NHSO₂ ;
- n est égal à 3.
ainsi que leur sels et esters d'alkyles en C₁-C₆ et l'ester benzylique.

6. Médicament, **caractérisé en ce qu'**il comprend un composé de formule (I) selon l'une quelconque des revendications 1 à 2, ou un hydrate ou un solvat du composé de formule (I).

7. Composition pharmaceutique, **caractérisée en ce qu'**elle comprend un composé de formule (I) selon l'une quelconque des revendications 1 à 2, ou un hydrate ou un solvat de ce composé, ainsi qu'au moins un excipient pharmaceutiquement acceptable.

8. Utilisation d'un composé de formule (I) selon l'une quelconque des revendications 1 à 2 pour la préparation d'un médicament destiné au traitement et à la prévention des maladies allergiques, des maladies inflammatoires, des maladies autoimmunes, de la douleur, des cancers, des maladies gastro-intestinales, de l'obésité.

9. Composé de formule (I) selon l'une quelconque des revendications 1 à 2 pour utilisation pour le traitement ou la prévention des maladies allergiques, des maladies inflammatoires, des maladies autoimmunes, de la douleur, des cancers, des maladies gastro-intestinales, de l'obésité.

## Claims

1. Compound corresponding to formula (I): 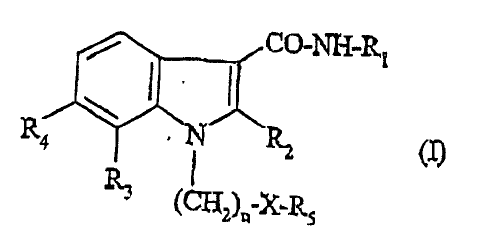 in which
- R₁ represents a 1,3,3-trimethylbicyclo[2.2.1]hept-2-yl or a bicyclo[3.2.1]oct-3-yl;
- R₂ represents hydrogen or a methyl;
- R₃ represents a chlorine, fluorine or bromine atom or a methyl group;
- R₄ represents a hydrogen, chlorine or fluorine atom or a methyl group;
- R₅ represents a methyl;
- X represents a sulfur atom, an -NHSO₂- group or an -SO₂- group;
- n is equal to 2 or 3,
and also in the form of a hydrate or of a solvate.

2. Compound according to Claim 1, **characterized in that** it is chosen from:
7-chloro-2-methyl-1-{3-[(methylsulfonyl)amino]-propyl}-N-[(1S)-endo-(1,3,3-trimethylbicyclo-[2.2.1]hept-2-yl)-1*H*-indole-3-carboxamide,
6,7-dichloro-2-methyl-1-{3-[(methylsulfonyl)-amino]propyl}-N-(1,3,3-trimethylbicyclo[2.2.1]hept-2-yl)-1*H*-indole-3-carboxamide, and
7-chloro-2-methyl-1-(3-(methylthio)propyl)-N-(1,3,3-trimethylbicyclo[2.2.1]hept-2-yl)-1*H*-indole-3-carboxamide;
and also in the form of a hydrate or of a solvate.

3. Process for preparing a compound of formula (I) according to either one of Claims 1 and 2, **characterized in that**:
a functional derivative of indole-3-carboxylic acid of formula: 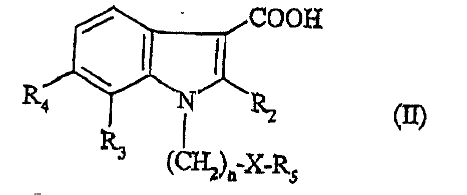
in which R₂, R₃, R₄, R₅, X and n are as defined for (I), is treated with an amine of formula R₁NH₂ (III).

4. Compound of formula: 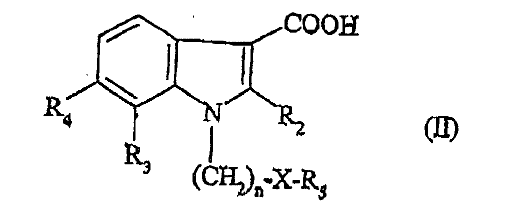 in which:
- R₂ represents a hydrogen atom or a methyl group;
- R₃ represents a chlorine, fluorine or bromine atom or a methyl group;
- R₄ represents a hydrogen, chlorine or fluorine atom or a methyl group;
- R₅ represents a methyl;
- X represents a sulfur atom, an -NHSO₂- group or an -SO₂- group;
- n is equal to 2 or 3;
and also their salts and C₁-C₆ alkyl esters and the benzyl ester.

5. Compound according to Claim 4 of formula (II) in which:
- R₂ represents a hydrogen atom or a methyl group;
- R₃ represents a chlorine or bromine atom;
- R₄ represents a hydrogen or chlorine atom or a methyl group;
- R₅ represents a methyl group;
- X represents a sulfur atom or an NHSO₂ group;
- n is equal to 3,
and also their salts and C₁-C₆ alkyl esters and the benzyl ester.

6. Medicinal product, **characterized in that** it comprises a compound of formula (I) according to either one of Claims 1 and 2, or a hydrate or a solvate of the compound of formula (I).

7. Pharmaceutical composition, **characterized in that** it comprises a compound of formula (I) according to either one of Claims 1 and 2, or a hydrate or a solvate of this compound, and also at least one pharmaceutically acceptable excipient.

8. Use of a compound of formula (I) according to either one of Claims 1 and 2, for preparing a medicinal product intended for the treatment and prevention of allergic disease, inflammatory diseases, autoimmune diseases, pain, cancers, gastrointestinal diseases and obesity.

9. Compound of formula (I) according to either one of Claims 1 and 2, for use in the treatment or prevention of allergic disease, inflammatory diseases, autoimmune diseases, pain, cancers, gastrointestinal diseases and obesity.

## Patentansprüche

1. Verbindung der Formel (I): 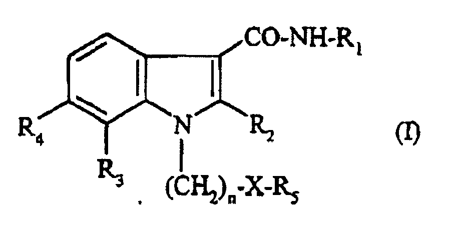 worin:
- R₁ für 1,3,3-Trimethylbicyclo[2.2.1]hept-2-yl oder Bicyclo[3.2.1]oct-3-yl steht;
- R₂ für Wasserstoff oder Methyl steht;
- R₃ für ein Chlor-, Fluor- oder Bromatom oder eine Methylgruppe steht;
- R₄ für ein Wasserstoff-, Chlor- oder Fluoratom oder eine Methylgruppe steht;
- R₅ für Methyl steht;
- X für ein Schwefelatom, eine -NHSO₂-Gruppe oder eine -SO₂-Gruppe steht;
- n gleich 2 oder 3 ist;
auch in Hydrat- oder Solvatform.

2. Verbindung nach Anspruch 1, **dadurch gekennzeichnet, daß** sie ausgewählt ist aus:
7-Chlor-2-methyl-1-{3-[(methylsulfonyl)amino]-propyl}-N-[(1S)-endo-(1,3,3-trimethylbicyclo-[2.2.1]hept-2-yl)-1H-indol-3-carboxamid,
6,7-Dichlor-2-methyl-1-{3-[(methylsulfonyl)-amino]propyl}-N-(1,3,3-trimethylbicyclo-[2.2.1]hept-2-yl)-1*H*-indol-3-carboxamid und 7-Chlor-2-methyl-1-(3-(methylthio)propyl)-N-(1,3,3-trimethylbicyclo[2.2.1]hept-2-yl)-1*H-*indol-3-carboxamid;
auch in Hydrat- oder Solvatform.

3. Verfahren zur Herstellung einer Verbindung der Formel (I) nach einem der Ansprüche 1 bis 2, **dadurch gekennzeichnet, daß** man:
ein funktionelles Derivat von Indol-3-carbonsäure der Formel: 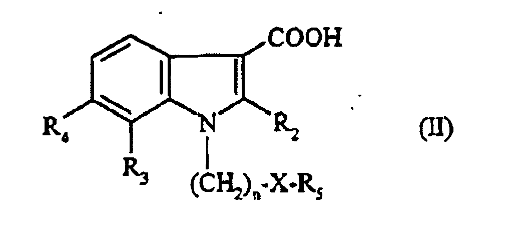
worin R₂, R₃, R₄, R₅, X und n die für (I) angegebene Bedeutung besitzen, mit einem Amin der Formel R₁NH₂ (III) behandelt.

4. Verbindung der Formel: 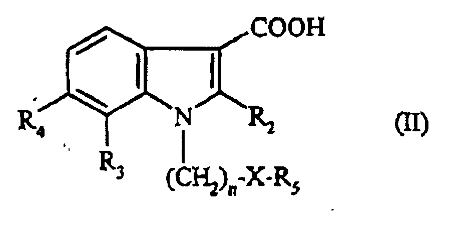 worin:
- R₂ für ein Wasserstoffatom oder eine Methylgruppe steht;
- R₃ für ein Chlor-, Fluor- oder Bromatom oder eine Methylgruppe steht;
- R₄ für ein Wasserstoff-, Chlor- oder Fluoratom oder eine Methylgruppe steht;
- R₅ für Methyl steht;
- X für ein Schwefelatom, eine -NHSO₂-Gruppe oder eine -SO₂-Gruppe steht;
- n gleich 2 oder 3 ist;
sowie deren Salze und C₁-C₆-Alkylester und der Benzylester.

5. Verbindungen der Formel (II) nach Anspruch 4, worin:
- R₂ für ein Wasserstoffatom oder eine Methylgruppe steht;
- R₃ für ein Chlor- oder Bromatom steht;
- R₄ für ein Wasserstoff- oder Chloratom oder eine Methylgruppe steht;
- R₅ für eine Methylgruppe steht;
- X für ein Schwefelatom oder eine NHSO₂-Gruppe steht;
- n gleich 3 ist;
sowie deren Salze und C₁-C₆-Alkylester und der Benzylester.

6. Arzneimittel, **dadurch gekennzeichnet, daß** es eine Verbindung der Formel (I) nach einem der Ansprüche 1 bis 2 oder ein Hydrat oder Solvat der Verbindung der Formel (I) umfaßt.

7. Pharmazeutische Zusammensetzung, **dadurch gekennzeichnet, daß** sie eine Verbindung der Formel (I) nach einem der Ansprüche 1 bis 2 oder ein Hydrat oder Solvat dieser Verbindung sowie mindestens einen pharmazeutisch unbedenklichen Trägerstoff umfaßt.

8. Verwendung einer Verbindung der Formel (I) nach einem der Ansprüche 1 bis 2 zur Herstellung eines Arzneimittels zur Behandlung und Prävention von allergischen Erkrankungen, entzündlichen Erkrankungen, Autoimmunerkrankungen, Schmerzen, Krebserkrankungen, Magen-Darm-Erkrankungen und Obesitas.

9. Verbindung der Formel (I) nach einem der Ansprüche 1 bis 2 zur Verwendung für die Behandlung oder Prävention von allergischen Erkrankungen, entzündlichen Erkrankungen, Autoimmunerkrankungen, Schmerzen, Krebserkrankungen, Magen-Darm-Erkrankungen und Obesitas.
